# EUROPEAN PATENT APPLICATION

(11) **EP 4 310 167 A1**
(43) Date of publication of application: **24.01.2024**
(21) Application number: 22186147.9
(22) Date of filing: 21.07.2022
(51) Int. Cl.: C12M 1/00, C12M 1/32

(54) **A REACTOR WITH PLATE-SHAPED CATALYTIC MEMBRANE FOR DIRECT CONVERSION OF MICROALGAE INTO BIOFUELS**

(71) Applicant: Socar Turkey Arastirma Gelistirme Ve Inovasyon A.S., Istanbul (TR)
(72) Inventor: Deliismail, Ozgun, Izmir (TR)
(74) Representative: Sevinç, Cenk

(57) **Abstract**

In the present invention, a reactor (2) for direct conversion of microalgae in a growth medium into biofuels, prevents energy consumption, reduce operating costs, reduce thermal stress, and provide simultaneous separation of polar and nonpolar compounds and salts is disclosed. The reactor (2) subject to the present invention comprises at least one compartment (1) containing a plate-shaped catalytic membrane (3) and two cells, a warm water inlet (5), a warm water outlet (6), a wet microalgae inlet (7), a liquid products and unconverted wet algae outlet (8).

## Description

### Technical Field of the Invention

The present invention relates to a reactor (2) with plate-shaped catalytic membrane (3) for direct conversion of microalgae in a growth medium into biofuels.

### Prior Art

Bioenergy is energy derived from biological sources. A biofuel could be any liquid fuel derived from biological materials, such as trees, agricultural wastes, crops, grass, animal waste or algae material. Biofuels can be produced from any carbon containing biological source and they are used globally, and biofuel industries are greatly expanding in Europe, Asia, and North and South America. They contain no sulfur and produce low carbon monoxide and toxic emissions. Biofuels are substitutes for conventional fossil fuels, such as petroleum, propane, coal, and natural gas. Common U.S. agricultural products including switchgrass and soybeans are specifically grown for biofuel production. Biofuels can reduce greenhouse gas emissions and increase energy security by providing an alternative to fossil fuels. By 2050, biofuels could reduce greenhouse gas emissions by 1.7 billion tons per year which is equivalent to more than 80% of current transportation-related emissions. Biofuels are classified based on their supply feedstock as primary, first generation, second generation, third generation and forth generation [1].

Primary biofuels, such as wood, require no additional processing prior to burning for energy. First-generation biofuels use food source materials, such as seeds and recycled food oil, to produce bio-oil and simple sugars that can be processed to produce fatty acid methyl esters (FAME) as biodiesel and glycerol, in addition to bioethanol and biobutanol, also biofuels. Seed oil was directly used in the early 20th century in diesel engines but It is now proven to be not suitable to use neat vegetable oil directly in vehicle combustion chambers because of the oil's high viscosity (28-40 mm²/s) compared to petrol diesel viscosity (3-4 mm²/s). This high viscosity leads to mainly engine coking. Second-generation biofuels use nonedible plant matter, such as wheat straw, to produce FAME, biofuels, and biobased chemicals. Third-generation biofuels use algae and, by several different means, to produce FAME, biofuels, in addition to several biochemicals. Fourth-generation biofuels also include biofuels that are produced by bioengineered organisms i.e. algae and cyanobacteria. Algae and cyanobacteria will use water, carbon dioxide, and solar energy to produce biofuels. This method of biofuel production is still at the research level. The biofuels that are secreted by the bioengineered organisms are expected to have higher photon-to-fuel conversion efficiency, compared to older generations of biofuels. One of the advantages of this class of biofuels is that the cultivation of the organisms that produce the biofuels does not require the use of arable land. The disadvantages include the cost of cultivating the biofuel-producing organisms being very high [2].

Algae, microalgae and macroalgae, can be grown on land in ponds or tanks and in the sea in the production of third or fourth generation biofuels. Algal fuels have high yields, can be grown with minimal impact on freshwater resources, can be produced using saline water and wastewater, have a high ignition point, and are biodegradable and relatively harmless to the environment if spilled. Production requires large amounts of energy and fertilizer, the produced fuel degrades faster than other biofuels, and it does not flow well in cold temperatures. By 2017, due to economic concerns, most efforts to produce fuel from algae have been abandoned or changed to other applications [3].

The necessity of harvesting microalgae from the nutrient medium in order to be used in biofuel and/or biochemical production is both technologically limited and costly. The number of microalgae in the growth medium varies between 3-10% by mass, and it is necessary to increase the number of microalgae up to about 20% by mass in order to be used in biofuel production in downstream streams with current technologies. While the separation of microalgae in the growth medium with current technologies, such as centrifugation, sedimentation, filtration, flocculation, etc., brings practical difficulties, and they also cause high operating costs. Thermochemical and biochemical processes are generally used in the conversion of harvested microalgae to biofuels. Among the thermochemical methods, pyrolysis is generally used in biofuel and/or biochemical production, and this process is based on the principle of oxygen-free decomposition of dry algae between 400-600°C temperatures. In addition, the liquefaction method, which is another approach used, is based on the conversion of wet algae, which is increased to 20% by mass by evaporation, into bio-oil at 300°C and 10 MPa conditions in a supercritical water environment. Thermochemical methods basically require high cost and high energy. Biochemical methods, such as anaerobic digestion and fermentation, especially used in methane and ethanol production, are not suitable for industrial applications because they require a long time and high water content, although they are less costly. Therefore, conversion of biomass and especially microalgae to biofuels and/or biochemicals with existing technologies is processes that require high energy and operating costs [4].

Batan et al. (2016) estimated using techno-economic analyses that growing, harvesting and lipid extraction of microalgae corresponds to 96% of the total operating cost. At the same time, they stated that the cost of dewatering and lipid extraction processes in the growth medium of microalgae covered approximately 33% and 32% of the total direct installed capital cost, respectively. Therefore, it is of great importance that the wet microalgae, which is 6% by mass in the seawater environment, is transformed directly into biofuels and biochemicals under low temperature, atmospheric pressure conditions and low reaction times by eliminating the procedures such as drying and harvesting.

In the prior art, KR101844783B1 patent relates to equipment and a method for producing bio-heavy fuel oil containing microalgae and sewage sludge as raw materials, and specifically, to the equipment and the method for producing the bio-heavy fuel oil using the microalgae and sewage sludge for producing the bio-heavy fuel oil by mixing the microalgae and sewage sludge well and by using a continuous alcohol supercritical process. Bio-oil is produced using supercritical conditions which is very high pressure and temperature, for example, at 217.8 atm and 373.9 °C for water. Using high temperature and pressure increases energy consumption. In this document, polar and non-polar products are not separated from the reaction medium at the same time. Since polar and non-polar products are not separated at the same time and also high temperature and pressure are used, energy consumption increases, and therefore, operational costs also increase.

In the prior art, there are problems, such as thermal stress; high energy consumption due to use of supercritical condition; high costs due to necessity of harvesting microalgae, separation of microalgae in the growth medium with current technologies, such as centrifugation, sedimentation, filtration, flocculation, etc. or cultivating the biofuel-producing organisms; non simultaneous separation of polar and nonpolar compounds and salts; unsuitable biochemical methods, such as anaerobic digestion and fermentation, especially used in methane and ethanol production because of requiring a long time and high water content in biofuel production. Because of these problems in prior art, developments in this technical field are required.

### Brief Description and Objects of the Invention

In the present invention, a reactor (2) for direct conversion of microalgae in a growth medium into biofuels, prevents energy consumption, reduce operating costs, reduce thermal stress, and provide simultaneous separation of polar and nonpolar compounds and salts is disclosed. The reactor (2) subject to the present invention comprises at least one compartment (1) containing two cells (4) and a plate-shaped catalytic membrane (3), a warm water inlet (5), a warm water outlet (6), a wet microalgae inlet (7), a liquid products and unconverted wet algae outlet (8). The number of compartments (1) varies according to the amount of water in the growth medium of the algae, production capacity and reaction time.

The most important object of the present invention is to directly convert microalgae grown in sea water in growth medium into biofuels. The reactor subject to the present invention comprises at least one compartment (1). In the compartment (1), wet microalgae enter the reactor (2), the wet microalgae react on the surface of a plate-shaped catalytic membrane (3). After the entry of wet microalgae in the compartment (1), water, polar products and salt pass to the other cell (4) in the first compartment (9) through the membrane inside the compartment (1). At the same time, the biofuels and the untransformed wet microalgae pass into the cell (4). Therefore, biofuel is produced directly by the reaction of wet microalgae on the surface of the plate-shaped catalytic membrane (3). The feature that enables the conversion of microalgae directly into biofuels is the catalyst. Catalysts have been synthesized to have acidic properties. The synthesized catalysts act as a bifunctional catalyst because both functions perform the hydrolysis reaction and break the carbon-carbon bonds in the lipid structures emerging from the microalgae structure as a result of the hydrolysis reaction. Thus, the lipids/proteins that emerge from the cell (4) by breaking down with the microalgae cell (4) walls with the hydrolysis reaction are broken down on the catalyst surface and converted into products (biofuel and biochemical) with different carbon distribution.

Another object of present invention is to prevent energy consumption in biofuel production. Temperature of compartment (1) in the reactor (2) is at least 80°C. In the invention, the reactor (2) is described that includes the conversion of microalgae to biofuels at temperatures of 150°C or below and at 1 atm conditions in the growth medium, as well as the simultaneous separation of the obtained polar and non-polar products from the reaction medium. Since biofuel is not produced in supercritical conditions such as 217.8 atm and 373.9°C, energy consumption is avoided in the present invention.

Another object of present invention is to reduce operating costs in biofuel production. The membrane in the invention provides in situ separation of polar and non-polar products, thus eliminating the need for extra costly units, such as removing the water in the microalgae growth medium. At the same time, any microalgae lipid extraction is not performed in the invention, and it is not based on the transesterification and/or esterification method. Therefore, the cost is low because the reactor (2) for use in biofuel production subject to the invention does not contain any extra processing steps.

Another object of present invention is to reduce thermal stress in biofuel production. The flows entering the compartment (1) in the reactor (2) described in the invention are in reverse flow to enable heat transfer to take place and to contribute to the reduction of thermal stress. With this configuration, the outlet temperature of the cold stream can approach the highest temperature of the hot stream. In addition, since the plate-shaped catalytic membrane (3) infrastructure is used, the driving force infrastructure is concentration, so reverse flow is used.

Another object of the invention is to provide simultaneous separation of polar and nonpolar compounds and salts in biofuel production. The most important feature of the membranes to be used in the invention is to ensure the passage of water and other polar products to the compartment (1) at high temperatures, and the use of water is used especially for the separation of salt and sugars because of its high solubility. Said temperature is less than 150°C

The present invention overcomes the problems present in the prior art by making developments in the reactor (2) in biofuel production. The present invention provides a reactor (2) which directly converts the microalgae in the growth medium, prevents energy consumption, reduces operating costs, reduces thermal stress, and provides simultaneous separation of polar and nonpolar compounds and salts in biofuel production.

### Brief Description of the Drawings

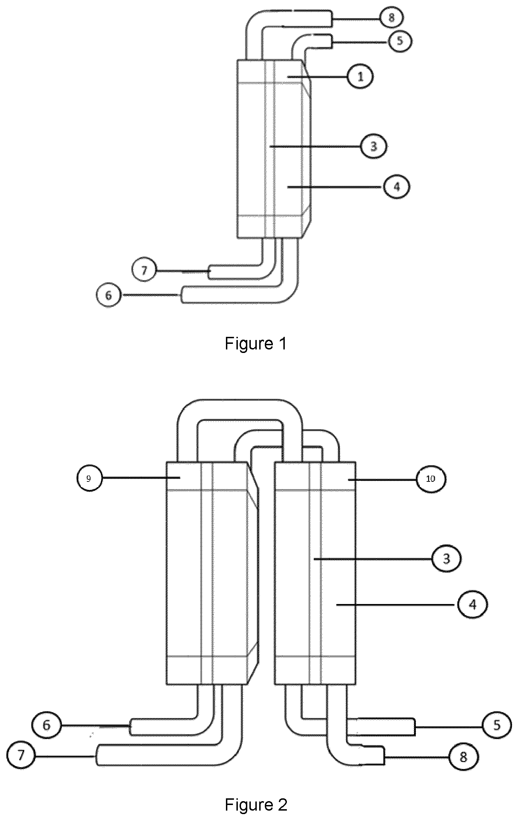

**Figure 1****.** Front view of the reactor (2) with one compartment.
**Figure 2****.** Front view of the reactor (2) with two compartments.

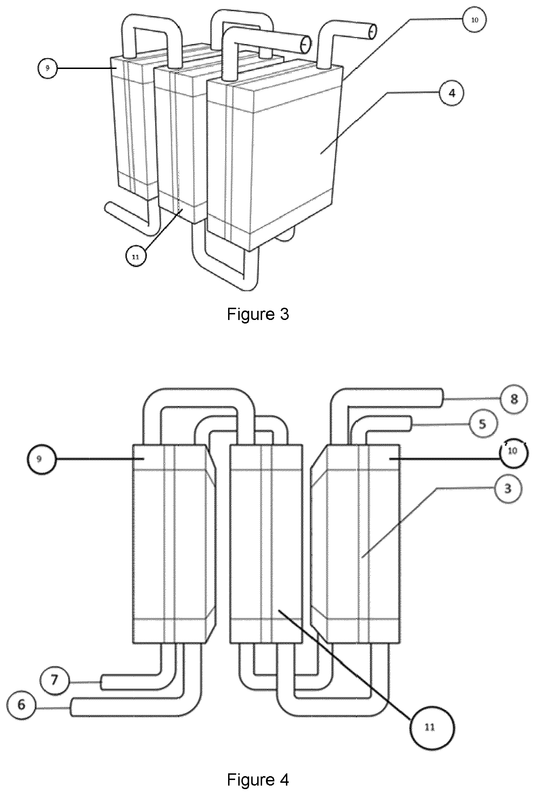

**Figure 3****.** Isometric view of the reactor (2) with more than two compartments.
**Figure 4****.** Front view of the reactor (2) with more than two compartments.

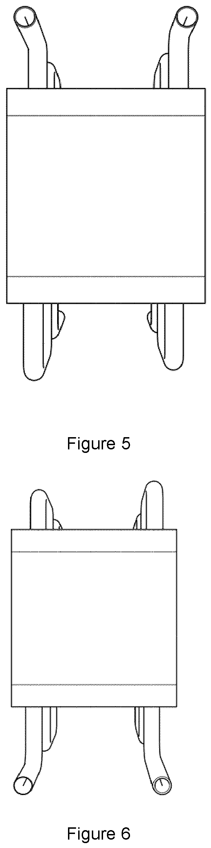

**Figure 5****.** Right view of the reactor (2) with more than two compartments.
**Figure 6****.** Left view of the reactor (2) with more than two compartments.

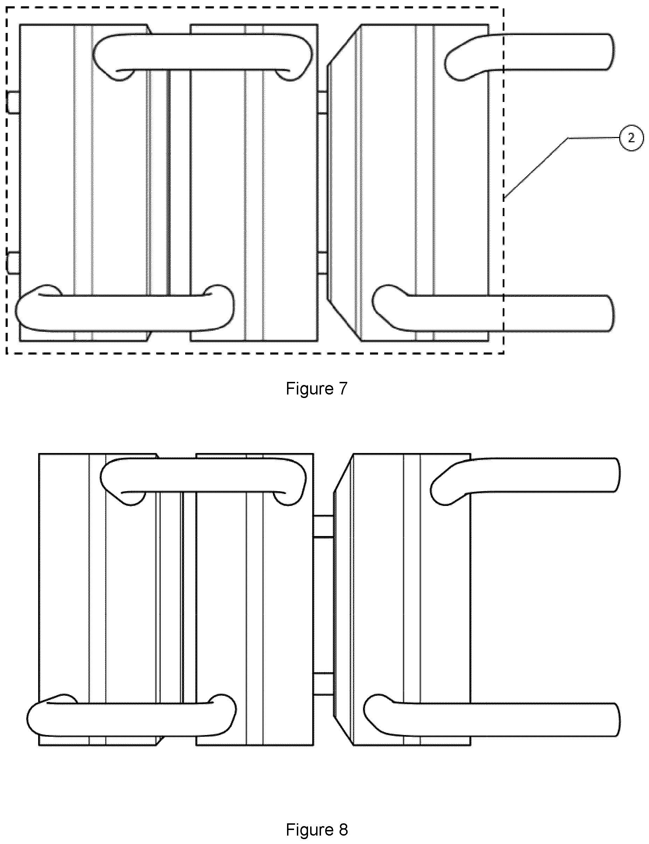

**Figure 7****.** Top view of the reactor (2) with more than two compartments.
**Figure 8****.** Bottom view of the reactor (2) with more than two compartments.

### Descriptions of References in Drawings

**1.** Compartment
**2.** Reactor
**3.** Plate-shaped catalytic membrane
**4.** Cell
**5.** Warm water inlet
**6.** Warm water outlet
**7.** Wet algae inlet
**8.** Liquid products and unconverted wet algae outlet
**9.** First compartment
**10.** Last compartment
**11.** Intermediate compartment.

### Detailed Description of the Invention

The present invention relates to a reactor (2) with plate-shaped catalytic membrane (3) for direct conversion of microalgae in a growth medium into biofuels which prevents energy consumption, reduce operating costs, reduce thermal stress, and provide simultaneous separation of polar and nonpolar compounds and salts. The growth medium varies according to the type of algae. In an embodiment of the invention, the growth medium comprises sea water and f/2 medium. Said f/2 medium comprises trace metals, vitamins, Na₂SiO₃.9H₂O, NaH₂PO₄.H₂O and NaNO₃. Said micro algae may be *Nannochloropsis oculato (N.oculata).*

In the present invention, a reactor (2) comprises at least one compartment (1) containing a plate-shaped catalytic membrane (3) which directly converts microalgae into biofuel and separates products and/or raw materials in a reaction medium and two cells (4), a warm water inlet (5) wherein warm water with a temperature between 80°C-90°C enters through, a warm water outlet (6) wherein warm water with a temperature between 80°C-90°C exits through, an wet microalgae inlet (7) wherein wet microalgae enter through, a liquid products and unconverted wet algae outlet (8) wherein liquid products and unconverted wet algae exit through. The plate-shaped catalytic membrane (3) converts microalgae into biofuel and separates products and/or raw materials in the reaction medium. Since the top of the plate-shaped catalytic membrane (3) is covered with the developed catalysts, it is used both for the reaction and for the separation of polar products and salt. The compartment (1) comprises a plate-shaped catalytic membrane (3) and two separate cells (4) in which the reaction products and the raw material, microalgae, in the environment decompose. The number of compartments (1) varies according to the amount of water in the growth medium of the algae, production capacity and reaction time. Thus, due to the parameters mentioned above (reaction time etc.), the reactor may have only one compartment or two compartments or more than one intermediate compartments (11) between the first compartment (9) and the last compartment (10). Especially, the number of compartments, which comprises a plate-shaped catalytic membrane (3) and two cells (4), is determined according to the reaction time to achieve a minimum of 96% algae conversion. The reactor (2) has a system using the plate-shaped catalytic membrane (3) infrastructure, and according to conventional methods, microalgae can be converted into biofuels such as sustainable jet fuel by using heterogeneous catalysts directly in low temperature and atmospheric pressure conditions in growth medium without harvesting and drying, and in-situ reaction of the products in water thanks to the membrane. The reactor (2) subject to the present invention is suitable for the use of microalgae grown in sea water and fresh water, and in fact, the salt in the sea water increases the microalgae transformation. The surface of the membranes is covered with catalysts synthesized using easily available, non-toxic and inexpensive materials. Said catalyst is alumina-silica supported nickel catalyst. Because of this ingredient, it is inexpensive and non-toxic.

In an embodiment of the invention, the reactor (2) comprises a compartment (1) containing a plate-shaped catalytic membrane (3) and two cells (4), a warm water inlet (5) wherein warm water with a temperature between 80°C-90°C enters through, a warm water outlet (6) wherein warm water with a temperature between 80°C-90°C exits through, an wet microalgae inlet (7) wherein wet microalgae enter through, a liquid products and unconverted wet algae outlet (8) wherein liquid products and unconverted wet algae exit through. Working method of the reactor (2) with one compartment comprises the following steps:
i. Entrance of the wet microalgae to one of the cells (4) through wet microalgae inlet (7), wherein the wet microalgae comprise a solid content between 2-10% in the growth medium,
ii. At the same time, entrance of warm water with a temperature between 80°C-90°C to the cell (4) through warm water inlet (5) in order to bring the reaction temperature to the desired point,
iii. After the entry of wet microalgae to the compartment (1), reacting wet microalgae on the plate-shaped catalytic membrane (3) surface,
iv. The passing of water, polar products and salt to the other cell (4) through the membrane inside the compartment (1),
v. After the end of the reaction, exit of liquid products and unconverted wet algae through the liquid products and unconverted wet algae outlet (8),
vi. At the same time, exit of warm water through the warm water outlet (6).

In another embodiment of the invention, the reactor (2) comprises two compartments (1) which are a first compartment (9) and a last compartment (10), a warm water inlet (5) wherein warm water with a temperature between 80°C-90°C enters through, a warm water outlet (6) wherein warm water with a temperature between 80°C-90°C exits through, a wet microalgae inlet (7) wherein wet microalgae enter through, a liquid products and unconverted wet algae outlet (8) wherein liquid products and unconverted wet algae exit through. Working method of the reactor (2) with two compartment comprises the following steps:
i. Entrance of the wet microalgae to one of the cells (4) through wet microalgae inlet (7), wherein the wet microalgae comprise a solid content between 2-10% in the growth medium,
ii. At the same time, entrance of warm water with a temperature between 80°C-90°C to the cell (4) through warm water inlet (5) in order to bring the reaction temperature to the desired point,
iii. After the entry of wet microalgae to the first compartment (9), reacting wet microalgae on the plate-shaped catalytic membrane (3) surface,
iv. The passing of water, polar products and salt to the other cell (4) through the membrane inside the first compartment (9),
v. At the same time, the passing of the biofuels produced by the untransformed wet microalgae into the cell (4) in the last compartment (10),
vi. In the last compartment (10), reacting wet microalgae on the plate-shaped catalytic membrane (3) surface,
vii. The passing of water, polar products and salt to the other cell (4) through the membrane inside the last compartment (10),
viii. Exit of liquid products and unconverted wet algae through the liquid products and unconverted wet algae outlet (8),
ix. Exit of warm water through the warm water outlet (6).

In another embodiment of the invention, the reactor (2) comprises at least three compartments (1) which are a first compartment (9), a last compartment (10) and at least one intermediate compartments (11), a warm water inlet (5) wherein warm water with a temperature between 80°C-90°C enters through, a warm water outlet (6) wherein warm water with a temperature between 80°C-90°C exits through, an wet microalgae inlet (7) wherein wet microalgae enter through, a liquid products and unconverted wet algae outlet (8) wherein liquid products and unconverted wet algae exit through. Working method of the reactor (2) with at least three compartments comprises the following steps:
i. Entrance of the wet microalgae to one of the cells (4) through wet microalgae inlet (7), wherein the wet microalgae comprise a solid content between 2-10% in the growth medium,
ii. At the same time, entrance of warm water with a temperature between 80°C-90°C to the cell (4) through warm water inlet (5) in order to bring the reaction temperature to the desired point,
iii. After the entry of wet microalgae to the first compartment (9), reacting wet microalgae on the plate-shaped catalytic membrane (3) surface,
iv. The passing of water, polar products and salt to the other cell (4) through the membrane inside the first compartment (9),
v. At the same time, the passing of the biofuels produced by the untransformed wet microalgae into the cell (4) in the intermediate compartment (11),
vi. In the intermediate compartment (11), reacting wet microalgae on the plate-shaped catalytic membrane (3) surface,
vii. The passing of water, polar products and salt to the other cell (4) through the membrane inside the intermediate compartment (11),
viii. After that, the passing of the biofuels produced by the untransformed wet microalgae into the cell (4) in the last compartment (10),
ix. In the last compartment (10), reacting wet microalgae on the plate-shaped catalytic membrane (3) surface,
x. The passing of water, polar products and salt to the other cell (4) through the membrane inside the last compartment (10),
xi. Exit of liquid products and unconverted wet algae through the liquid products and unconverted wet algae outlet (8),
xii. Exit of warm water through the warm water outlet (6).

In another embodiment of the invention, when number of compartments is odd, the warm water inlet (5) and the liquid products and unconverted wet algae outlet (8) are located in the upper right corner of the reactor (2), and the wet algae inlet (7) and warm water outlet (6) are located at the lower-left corner of the reactor (2) to create reverse flow. On the other hand, when number of compartments is even, the warm water inlet (5) and the liquid products and unconverted wet algae outlet (8) are located in the lower right corner of the reactor (2), and the wet algae inlet (7) and warm water outlet (6) are located at the lower-left corner of the reactor (2) to create reverse flow. Reverse flow provides to enable heat transfer to take place and to contribute to the reduction of thermal stress.

The plate-shaped catalytic membrane (3) in the reactor (2) comprises two separate cells (4) from each other. Wet microalgae enter one of the cells (4) at the end points through wet microalgae inlet, wherein the wet microalgae comprise a solid content between 2-10% in the growth medium. At the same time, warm water with a temperature between 80°C-90°C enters the cell (4) of the last compartment (10) in order to bring the reaction temperature to the desired temperature levels. The flow of wet microalgae and warm water enter the compartments (1) in reverse directions. In the first compartment (9), wet microalgae enter the reactor (2), the wet microalgae react on the surface of the plate-shaped catalytic membrane (3). After the entry of wet microalgae in the first compartment (9), water, polar products and salt pass to the next cell (4) through the membrane inside the compartments (1). At the same time, the biofuels produced by the wet microalgae pass into the cell (4) in the next compartment (1). Here, the reaction on the plate-shaped catalytic membrane (3) surfaces the separation of the products with the membrane continues. This process continues in proceeding cells (4) until the number of microalgae is highly reduced. The reactor (2) is a continuous process and not a batch process. The reactor (2) must be continuously fed with microalgae in the growth medium, which is the raw material of the process, and warm water within the above-mentioned temperature range.

The cell wall of *Nannochloropsis oculato (N.oculata)* microalgae is composed of cellulose. Therefore, catalysts have been synthesized to have acidic properties. The synthesized catalysts act as a bifunctional catalyst because both functions perform the hydrolysis reaction and break the carbon-carbon bonds in the lipid structures emerging from the microalgae structure as a result of the hydrolysis reaction. Thus, with the hydrolysis reaction, the lipids/proteins that emerged from the cell (4) by breaking down with the microalgae cell (4) walls were broken on the catalyst surface and converted into products, such as biofuel or biochemical with different carbon distribution. Microalgae, whose sizes vary between 3-6 µm, cannot penetrate into the pores of the catalysts; thus, reactions occur on the surface of the catalyst particles in the first stage. As the production of biofuels/biochemicals from microalgae without harvesting and drying within the scope of the invention takes place on heterogeneous catalysts covered membrane, it is another important feature that the limitations arising from mass transfer are reduced as compared to batch reactors.

GC/MS and HPLC analyzes of the products obtained at the exit of the reactor (2) were performed. GC/MS and HPLC results are shown in Table 1 and Table 2.

**Table 1. GC-MS analysis results of non-polar products obtained from the reactor (2).**

| **Non-polar products** | **Peak Area (%)** |
|---|---|
| C₉H₁₂ (Benzene, 1,3,5-trimethyl) | 30.1 |
| C14H30 (Tetradecane) | 5.5 |
| C₁₆H₃₄ (Hexadecane) | 13.3 |
| C2oH42 (Eicosane) | 10.2 |
| C18H38 (Octadecane) | 12.7 |
| C18H36O2 (Hexadecenoic acid, ethyl ester) | 16.5 |
| C₂₀H₄₀O₂ (Octadecanoic acid, ethyl ester) | 11.8 |

**Table 2. HPLC analysis results of polar products obtained from the reactor (2).**

| **Polar products** | **Concentration (mg/L)** |
|---|---|
| Glucose | 145 |
| Glycerol | 81 |
| Arabinose | 36 |

According to Table 1 and Table 2, the carbon distribution of the output products ranges from C9 to C20, and most products are linear. Obtained fatty acids and their esters also show that there are transesterification reactions occurring. The points to be considered during the use of the reactor (2) subject to the invention are as follows:
- The temperature of each compartment (1) in the reactor (2) should be at least 80°C and maximum 150°C. In the preferred embodiment of the invention, the reaction temperature should be 80°C. Membranes withstand temperatures of 120°C and above. However, the product distribution will vary, as carbon fractures will be higher above 80°C.
- Microalgae in the growth medium, which is the raw material of the process, and warm water in the above-mentioned temperature range should be fed to the reactor (2).
- Each compartment (1) in the reactor (2) should be brought to steady state conditions.
- The control of the obtained product should be followed by taking a sample every three hours by GC-MS.
- Plate-shaped catalytic membranes (3) in the reactor (2) should be checked every 3-5 years.

### REFERENCES

**[1]** Roberts, L., & Patterson, T. (2014). Biofuels. Encyclopedia of Toxicology, 469-475. https://doi.org/10.1016/b978-0-12-386454-3.01054-x
**[2]** Dahman, Y., Syed, K., Begum, S., Roy, P., & Mohtasebi, B. (2019). Biofuels. Biomass, Biopolymer-Based Materials, and Bioenergy, 277-325. https://doi.org/10.1016/b978-0-08-102426-3.00014-x
**[3]** Biofuels: The Promise of Algae. (2021). BIO. https://archive.bio.org/articles/biofuels-promise-algae#:%7E:text=The%20carbohydrates%20(sugars)%20from%20algae,c ombined%20heat%20and%20power%20generation.
**[4]** Behera, S., Singh, R., Arora, R., Sharma, N. K., Shukla, M., & Kumar, S. (2015). Scope of Algae as Third Generation Biofuels. Frontiers in Bioengineering and Biotechnology, 2. https://doi.org/10.3389/fbioe.2014.00090

## Claims

1. A reactor (2) with a plate-shaped catalytic membrane (3) for conversion of microalgae in a growth medium into biofuels **characterized by** comprising,
• at least one compartment (1) comprising a plate-shaped catalytic membrane (3) which directly converts microalgae into biofuel and separates products and/or raw materials in a reaction medium and two cells (4),
• a warm water inlet (5), wherein warm water with a temperature between 80°C-90°C enters through,
• a warm water outlet (6), wherein warm water with a temperature between 80°C-90°C exits through,
• a wet microalgae inlet (7), wherein wet microalgae enter through,
• a liquid products and unconverted wet algae outlet (8), wherein wet microalgae exit through.

2. The reactor (2) according to claim 1, the reactor (2) comprises one compartment (1).

3. The reactor (2) according to claim 1, the reactor (2) comprises two compartments (1) having a first compartment (9) and a last compartment (10).

4. The reactor (2) according to claim 3, the reactor (2) further comprises at least one intermediate compartment (11) between a first compartment (9) and a last compartment (10).

5. The reactor (2) according to claim 1-4, the warm water inlet (5) and the liquid products and unconverted wet algae outlet (8) are located in the upper right corner of the reactor (2), and the wet algae inlet (7) and warm water outlet (6) are located at the lower-left corner of the reactor (2) to create reverse flow when number of compartments is odd.

6. The reactor (2) according to claim 1-4, the warm water inlet (5) and the liquid products and unconverted wet algae outlet (8) are located in the lower right corner of the reactor (2), and the wet algae inlet (7) and warm water outlet (6) are located at the lower-left corner of the reactor (2) to create reverse flow when number of compartments is even.

7. The reactor (2) according to claim 1, wherein the surface of the membrane is covered with catalysts.

8. The reactor (2) according to claim 7, wherein the catalysts is alumina-silica supported nickel catalyst.

9. The reactor (2) according to claim 1, wherein the wet microalgae comprise a solid content between 2-10% in the growth medium.

10. The reactor (2) according to claim 9, wherein the growth medium comprises sea water and f/2 medium.

11. The reactor (2) according to claim 10, wherein f/2 medium comprises trace metals, vitamins, Na₂SiO₃.9H₂O, NaH₂PO₄.H₂O and NaNO₃.

12. **The** reactor (2) according to claim 1, wherein the microalgae is *Nannochloropsis oculato (N. oculata).*

13. Working method of the reactor (2) according to claim 2 **characterized by** comprising the following steps:
i. Entrance of the wet microalgae to one of the cells (4) through wet microalgae inlet (7), wherein the wet microalgae comprise a solid content between 2-10% in the growth medium,
ii. At the same time, entrance of warm water with a temperature between 80°C-90°C to the cell (4) through warm water inlet (5) in order to bring the reaction temperature to the desired point,
iii. After the entry of wet microalgae to the compartment (1), reacting wet microalgae on the plate-shaped catalytic membrane (3) surface,
iv. The passing of water, polar products and salt to the other cell (4) through the membrane inside the compartment (1),
v. After the end of the reaction, exit of liquid products and unconverted wet algae through the liquid products and unconverted wet algae outlet (8),
vi. At the same time, exit of warm water through the warm water outlet (6).

14. Working method of the reactor (2) according to claim 3 **characterized by** comprising the following steps:
i. Entrance of the wet microalgae to one of the cells (4) through wet microalgae inlet (7), wherein the wet microalgae comprise a solid content between 2-10% in the growth medium,
ii. At the same time, entrance of warm water with a temperature between 80°C-90°C to the cell (4) through warm water inlet (5) in order to bring the reaction temperature to the desired point,
iii. After the entry of wet microalgae to the first compartment (9), reacting wet microalgae on the plate-shaped catalytic membrane (3) surface,
iv. The passing of water, polar products and salt to the other cell (4) through the membrane inside the first compartment (9),
v. At the same time, the passing of the biofuels produced by the untransformed wet microalgae into the cell (4) in the last compartment (10),
vi. In the last compartment (10), reacting wet microalgae on the plate-shaped catalytic membrane (3) surface,
vii. The passing of water, polar products and salt to the other cell (4) through the membrane inside the last compartment (10),
viii. Exit of liquid products and unconverted wet algae through the liquid products and unconverted wet algae outlet (8),
ix. Exit of warm water through the warm water outlet (6).

15. Working method of the reactor (2) according to claim 4 **characterized by** comprising the following steps:
i. Entrance of the wet microalgae to one of the cells (4) through wet microalgae inlet (7), wherein the wet microalgae comprise a solid content between 2-10% in the growth medium,
ii. At the same time, entrance of warm water with a temperature between 80°C-90°C to the cell (4) through warm water inlet (5) in order to bring the reaction temperature to the desired point,
iii. After the entry of wet microalgae to the first compartment (9), reacting wet microalgae on the plate-shaped catalytic membrane (3) surface,
iv. The passing of water, polar products and salt to the other cell (4) through the membrane inside the first compartment (9),
v. At the same time, the passing of the biofuels produced by the untransformed wet microalgae into the cell (4) in the intermediate compartment (11),
vi. In the intermediate compartment (11), reacting wet microalgae on the plate-shaped catalytic membrane (3) surface,
vii. The passing of water, polar products and salt to the other cell (4) through the membrane inside the intermediate compartment (11),
viii. After that, the passing of the biofuels produced by the untransformed wet microalgae into the cell (4) in the last compartment (10),
ix. In the last compartment (10), reacting wet microalgae on the plate-shaped catalytic membrane (3) surface,
x. The passing of water, polar products and salt to the other cell (4) through the membrane inside the last compartment (10),
xi. Exit of liquid products and unconverted wet algae through the liquid products and unconverted wet algae outlet (8),
xii. Exit of warm water through the warm water outlet (6).

16. Working method of the reactor (2) according to claim 13-15, wherein the microalgae is *Nannochloropsis oculato (N. oculata).*

17. Working method of the reactor (2) according to claim 13-15, wherein the surface of the membrane is covered with catalysts.

18. Working method of the reactor (2) according to claim 17, wherein the catalysts is alumina-silica supported nickel catalyst.

19. Working method of the reactor (2) according to claim 13-15, wherein the wet microalgae comprise a solid content between 2-10% in the growth medium.

20. The reactor (2) according to claim 19, wherein the growth medium comprises sea water and f/2 medium.

21. The reactor (2) according to claim 20, wherein f/2 medium comprises trace metals, vitamins, Na₂SiO₃.9H₂O, NaH₂PO₄.H₂O and NaNO₃.
